Europäisches Patentamt

(19) European Patent Office

Office européen des brevets

(11) Publication number: **0 014 218**
**B1**

(12) **EUROPEAN PATENT SPECIFICATION**

(45) Date of publication of patent specification: **28.12.83**

(21) Application number: **79100020.1**

(22) Date of filing: **04.01.79**

(51) Int. Cl.³: **B 01 J 37/00,**
**B 01 J 23/86, C 07 C 5/333**

(54) Unsupported fixed-bed catalyst tablet and method for making same.

(43) Date of publication of application:
**20.08.80 Bulletin 80/17**

(45) Publication of the grant of the patent:
**28.12.83 Bulletin 83/52**

(84) Designated Contracting States:
**DE FR GB IT NL**

(56) References cited:
**US - A - 3 862 207**
**US - A - 4 124 699**

**CHEM. ENG. PROGRESS, vol. 70, no. 11, page 80, November 1974, VOLZ: "Spray Drying and Customized Catalyst"**
**Ullmanns Encyklopädie der techn. Chemie, 4. Aufl., Band 13, S. 564**

(73) Proprietor: **THE DOW CHEMICAL COMPANY**
**Dow Center 2030 Abbott Road Post Office Box 1967**
**Midland Michigan 48640 (US)**

(72) Inventor: **Castor, William Mack**
**310 South Yaupon**
**Clute Brazoria Texas (US)**
Inventor: **Menegos, Pete James**
**203 Oyster Bend Lane**
**Lake Jackson Brazoria Texas (US)**

(74) Representative: **Casalonga, Alain et al,**
**BUREAU D.A. CASALONGA OFFICE JOSSE & PETIT Baaderstrasse 12-14**
**D-8000 München 5 (DE)**

**0014218**

Unsupported fixed-bed catalyst tablet and method for making same

Non-supported, fixed-bed catalyst pellets are used in many chemical processes. Among these are processes for dehydrogenation, hydrogenation, and oxidation of organics. Dehydrogenation processes using such fixed-bed, non-supported catalysts include the dehydrogenation of ethylbenzene to produce styrene, of ethyl toluene to produce vinyl toluene, and of butane or butene to produce butadiene.

Hydrogenation processes which use such fixed-bed, non-supported catalysts include such processes as that for producing saturated hydrocarbons from olefins.

Oxidation processes which use such fixed-bed, non-supported catalyst pellets include such processes as those for making aldehydes and acids from saturated and unsaturated hydrocarbons, e.g., acrylic acid from propylene.

For the sake of conciseness, the word "pellets" is hereinafter used as a generic term to include those spray dried tablets made by the process of this invention as well as other forms of pellets made by such processes as extrusion, i.e., extrudates. "Tablet" is defined to be any pellet made by the process of this invention.

In the past, improvements in the catalytic performance and/or crush strength of non-supported catalyst pellets for the processes referred to above, have generally been obtained by changing the formulation of components. Typically such non-supported catalyst pellets are made by extruding a paste of components through cylindrical dies to form moist cylinders having a diameter of from about 1/16 inch to about 1/2 inch (0.16—1.27 cm). As these cylinders exit from the extruder they are usually either chopped into predetermined lengths or allowed to break of their own weight, forming cylindrical pellets (extrudates) having a length of from about 1/8 inch (0.32 cm) to about one inch (2.54 cm).

Methods other than extrusion have been taught for forming non-supported catalyst pellets. For example U.S. Patent 1,680,807 (August 14, 1928) teaches the formation of catalyst pellets by compression of a multiplicity of fine particles of catalytic material, which particles are not naturally coherent in the dry state. Spray drying of fluid bed catalysts is known. See U.S. Patents 2,768,145 (Tongue et al. 1956) and 1,680,807 (Scultze 1928). Spray drying catalyst materials on catalyst carriers or supports is known. See U.S. Patent 2,435,379 (Archibald 1948).

The present invention is directed to a method for making an unsupported fixed-bed catalyst tablet, said method characterized by

(a) forming microspheres from a slurry of catalyst source constituents of the oxides of iron, potassium, and chromium by spray drying a slurry containing these catalyst source constituents;

(b) forming a catalyst tablet by subjecting a preselected amount of the microspheres to a compressive pressure of at least $208 \times 10^5$ Pa ($212$ kg/cm$^2$) and

(c) calcining the tablet at a temperature greater than 500°C.

For some unknown reason this combination of steps produces a catalyst tablet having superior catalytic performance and/or crush strength qualities.

The present invention is also directed to catalyst tablets made by the process described immediately above.

It should be noted that the phrase "a slurry of catalyst source components" is used above in the spray drying step as opposed to "a slurry of catalyst components". This is done to point out that sometimes the constituents used in making up the slurry for spray drying are not the same components as those found in the final formulation of the catalyst tablets to be used. Often after adding the constituents together, or in subsequent steps in making the catalyst pellets, chemical changes occur in these constituents. Thus different constituents other than those started with often appear in the final catalyst pellet formulation. Of course, there are also catalyst slurry formulations which are not changed. This invention is applicable to both. Hence, as used hereinafter the phrase "catalyst source consitituents" is defined to mean not only those constituents which appear in one form in the slurry and another form in the finished catalyst tablet, but also to those constituents that appear in the same form in both the slurry and the finished tablet. For example $K_2CO_3$ is a source for $K_2O$ if the $K_2CO_3$ is converted to $K_2O$. Also $K_2O$ itself is a source of $K_2O$ by this definition.

The tablets made by the claimed process are useful in various organic chemical processes, including dehydrogenation, hydrogenation, and oxidation processes.

In many applications the catalyst tablets must be further treated to be useful. For example, dehydrogenation catalyst tablets often must be calcined, while oxidation catalyst tablets often must be heated in the presence of hydrogen or a hydrogen source.

The method of making these tablets has found use in making fixed-bed, non-supported, dehydrogenation catalyst tablets for use in the known process of dehydrogenation of alkyl aromatics, e.g. those aromatics having alkyl groups containing from two to eight carbon atoms, and particularly dehydrogenation of ethylbenzene to styrene. This process comprises passing at an elevated temperature, ethylbenzene mixed with steam over catalyst tablets, said catalyst tablets containing at least oxides of iron, potassium and chromium.

**0014218**

To specifically illustrate the process by which these new catalyst tablets are made as well as the unexpected improvement in catalytic performance and crush strength of the tablets made by this process, the discussion and examples given below refer to a catalyst tabletting process, and the tablets made by this process, which are used in the dehydrogenation of ethylbenzene to produce styrene.

Typically, sytrene is made in a dehydrogenation reactor by passing ethylbenzene and steam at an elevated temperature through a bed of catalyst pellets. These pellets are comprised of oxides of iron, potassium and chromium.

In the past these pellets have usually been formed by a process comprising:

(a) forming a paste comprised of water, lubricant, binders and catalyst source constituents of the oxides of iron, potassium, and chromium;
(b) extruding the paste into cylindrical pellets having a diameter of from about 1/8 inch (0.32 cm) to about 3/16 inch (0.48 cm) and a length of from about 1/8 inch (0.32 cm) to about 3/4 inch (1.90 cm); and
(c) calcining the pellets at a temperature of from about 500°C to about 1200°C.

The step of the method of the invention will now be elaborated upon in the following discussion.

Slurry formation

In forming a slurry suitable for spray drying, any method can be used which gets the catalyst formation components into an aqueous solution, aqueous suspension, or both.

The amount of water present in the slurry is not critical. This amount depends only on the operating capabilities of the particular spray dryer used. A slurry containing from 20 weight percent to 70 weight percent water is found satisfactory for nearly all spray dryers. A range of 40 weight percent to 60 weight percent water is preferable for most disk type spray dryers.

The oxides of iron, chromium, and potassium which are found to be necessary in the final catalyst tablet can be provided in other chemical forms in the slurry, such as, for example, potassium carbonate, potassium dichromate and ferric oxide. These other · chemical forms, however, must be convertible to the oxides during the remainder of the process of making the tablets. The calcining step is usually the step in which these other forms of iron, potassium, and chromium are converted to the oxides, $Fe_2O_3$, $Cr_2O_3$, and $K_2O$. It is immaterial in what form the elements of iron, potassium, chromium, and oxygen occur in the slurry preparation step so long as they can be changed to the oxides themselves in the finished catalyst tablet.

In preparing pellets by the old method of extrusion (i.e., preparing extrudates), it is known that the water-insoluble constituents of the slurry should be ground or otherwise converted into powdered particles. Usually this powder is available commercially. The same water-insoluble powdered ingredients used in the process of producing extrudates are used in the process of this invention. For the comparative runs given below, for each diameter pellet of each formulation, the ingredients used were from the same source. This was done so that there would be no variation in the test results due to variations in the source materials.

Spray drying

Spray drying is a process long known as a process for producing very small, discontinuous, spherical particles from a slurry feed, the particles being microspheres usually having a diameter no greater than about 500—1,000 $\mu$m for most formulations from most spray dryers. For a general description of spray dryers and their operation, see *Perry's Chemical Engineers' Handbook*, John H. Perry, editor, Third Edition, McGraw-Hill Book Co., Inc., New York, Toronto, and London, pages 838—848 (1950).

Spray dryers are classified into three general categories, centrifugal disk, pressure nozzles, and two-fluid nozzles. Any of these are suitable for use in the process of this invention, but the disk type is preferred for large-scale commercial operations.

Neither the moisture content nor the particle size distribution of the microspheres made by the spray drying step of this invention are critical limitations. However, there are practical limitations that the particular spray dryer being used imposes upon these two parameters, and the microspheres' diameters should not be of the same order of magnitude as the finished non-supported catalyst tablets desired to be produced.

Having the diameter of the spray dried microspheres approach the size of the finished tablets poses no problem generally. This is so because the diameter or size of the smallest tablet contemplated by this invention is about 0,16 cm (1/16 inch) while the largest microspheres usually produced by commercial spray dryers are on the order of only about 500—1,000 $\mu$m. This size microsphere is within the operable limits for making the tablets contemplated by this invention. It was found that better catalytic performance of the catalyst tablets was achieved when the diameter of about 80 percent of the microspheres was greater than 20 $\mu$m. It is preferable to have a particle size distribution of the spray dried microspheres such that about 80 percent of the microspheres have diameters which lie in a range of from 20 $\mu$m to 200 $\mu$m, and more preferable if this range is from 20 $\mu$m to 100 $\mu$m.

3

**0 014 218**

Smaller microspheres caused the equipment used in the tabletting step to bind and exhibit extreme wear. The larger sizes caused no perceptible problems.

Variations in moisture content of the spray dried microspheres was not critical for the catalytic performance of the finished tablets. Moisture content of the microspheres had more of an effect on the operation of the particular spray dryer and auxiliary equipment used. A moisture content of from zero weight percent to 5.0 weight percent produced catalytic tablets which were satisfactory in their catalytic performance and crush strength as well as for operation of the equipment. Large moisture contents caused poor flow properties.

Compression into tablets

After spray drying the slurry into microspheres, the next step in the process of this invention is to form relatively large tablets from these microspheres by subjecting predetermined amounts of these microspheres to a compressive pressure. This compressive pressure is not critical, but should be great enough to produce tablets having sufficient crush strength to withstand the physical loads and attrition to which they will ultimately be subjected. Usually such compressive pressures are best achieved in punch and die equipment.

As a general rule, the greater the tabletting pressure used, the greater will be the crush strength of the tablets. Tablets with adequate strength were made using a compressive tabletting pressure as low as $208 \times 10^5$ Pa ($212$ kg/cm$^2$). It is generally preferred to use a compressive pressure of greater than $1038 \times 10^5$ Pa ($1060$ kg/cm$^2$). It is more preferred to use a compressive pressure of from $1519 \times 10^5$ Pa ($1550$ kg/cm$^2$) to $3116,4 \times 10^5$ Pa ($3180$ kg/cm$^2$) and most preferable to use a compressive pressure of from $1940,4 \times 10^5$ Pa to $2626,4 \times 10^5$ Pa ($1980$—$2680$ kg/cm$^2$). The higher pressures of these latter two pressure ranges are chosen as a compromise between acceptable tablet crush strengths and catalytic performance and the pressure operating limits of the equipment used. No upper limit was found for the compressive pressure, however. Compressive pressures greater than $8976,8 \times 10^5$ Pa ($9160$ kg/cm$^2$) were used in a laboratory test run which produced very good tablets, but known commercial equipment was not available for large-scale manufacture of tablets made with this large a pressure.

Subjection of the spray dried material to compressive pressures greater than from about $208 \times 10^5$—$1038,8 \times 10^5$ Pa ($212$—$1060$ kg/cm$^2$) is herein also referred to as "tabletting". The pellets formed by such tabletting are herein also referred to as "tablets" to distinguish them from those pellets formed by extrusion, herein also referred to as extrudates.

In the tabletting step of the process, the molds or dies are chosen with shapes such that the tablets formed will have the desired size and shape.

The most preferred shape for fixed-bed, non-supported catalyst pellets produced by any method is spherical. Adequate dies to produce such spherical shapes, however, were not readily available at the time the experiments which follow herein were conducted. Thus no examples are shown for making spherical shaped fixed-bed, self-supporting catalyst tablets. This shape, however, is certainly within the scope of this invention as are other non-cylindrical shaped pellets, such as ellipsoids, cubes, star shapes, frustoconical shapes and hollow cylinders.

Generally cylindrically shaped tablets are easier to make and those with convex ends are preferable. There is no critical size limit for these tablets. Catalytic performance tends to fall off as the diameter of the tablets varies from about $0,48$ cm ($3/16$ inch) in the styrene reactors tested. The diameter of these cylindrical tablets can be from $0,16$ cm ($1/16$ inch) to $2,54$ cm (one inch). Preferably the diameter is from $0,32$ cm ($1/8$ inch) to $1,27$ cm ($1/2$ inch) with a more preferred diameter being from $0,32$ cm ($1/8$ inch) to $0,64$ cm ($1/4$ inch). The most preferred diameter is $0,48$ cm ($3/16$ inch). The length of the cylinders can be from $0,16$ cm ($1/16$ inch) up to one inch ($2.54$ cm). It has been found, however, that less attrition and mechanical breakage of the tablets occur if the tablets are made so that their length is approximately the same as their diameter.

Calcining

Following the compression of the microspheres to form tablets, these tablets are usually calcined. Typically calcining is carried out at a temperature of from 500°C to 1200°C for a time of from 1 or 2 to 20 hours. Calcining can be carried out in a special calcining chamber or in the reactor itself. This calcining step for the tablets formed by the method of this invention is carried out in virtually the same manner as has heretofore been done for the calcining of extrudates.

Examples of making spray dried tablets by the method of this invention

A slurry was made as follows: $5330$ l ($1411$ US gal) of water were added the following water-soluble solid constituents in the amounts given: $2540$ kg ($5600$ lb) of $K_2CO_3 \cdot 3/2H_2O$, $381$ kg ($839,5$ lb) of $K_2Cr_2O_7$, and $381$ kg ($839,5$ lb) of $V_2O_5$. To this solution was added the following water-insoluble solid components in powdered form in the amounts given and in particle size distribution given: $6610$ kg ($14,573$ lb) of $Fe_2O_3$ powdered particles ranging in size from 3 $\mu$m to 49 $\mu$m $2670$ kg ($5883$ lb) of $Fe_2O_3 \cdot H_2O$ powdered particles ranging in size from 2 $\mu$m to 52 $\mu$m, $395$ kg ($869$ lb) of Portland cement ranging in size from 2 $\mu$m to 55 $\mu$m, $1943$ kg ($4278$ lb) of methyl cellulose (a binder) powdered

4

# 0 014 218

particles ranging in size from 3 μm to 250 μm; and 1880 kg (4145 lb) of graphite powdered particles ranging in size from 1 μm to 13 μm.

Additional water was added to the above slurry so there was a solids to water ratio of 0.94:1 in the resulting slurry.

This suspension was then fed as the feed to the feed inlet pump of a centrifugal rotating disk type spray dryer at a temperature of 30°C. The model used had an atomizing disk wheel 12,7 cm (5 inches) in diameter which was located near the top of the drying chamber of the spray dryer. The drying chamber was conical in shape and supported vertically with the larger portion above the smaller portion. Its largest diameter was 2,14 m (7 feet) and its height was 3,66 m (12 feet). The flow of the drying air was concurrent to the downward falling of the microspheric particles.

The rate of the feed to the atomizer disk was 2,91 l/m (0.77 US gal/min). The disk was rotated at a speed of 21,600 rpm. The inlet temperature of the drying air was 260°C while its outlet temperature was 149°C.

The dried microspheres were exhausted from the spray drying chamber of the spray dryer into its bag filter collector. The microspheres were observed to be both of the ruptured surface and smooth surface type. They had a particle size distribution of from 5 μm to 27 μm diameter with moisture of less than 1 weight percent.

The microspheres were then fed to a tabletting machine having 0,51 cm (0.200 inch) inside diameter dies and were compressed with punches at a pressure of 2570 kg/cm² (36,400 psi).

The tablets produced by the tabletting machine were cylindrical in shape. They were 0,52 cm (0,210 inch) in diameter and long 0,46—0,74 cm (0,180 to 0.290 inch). They had an average crush strength of 13,2 kg (29 lb), as measured by a hardness tester instrument.

The tablets were then calcined in a kiln at temperatures ranging from 683°C to 732°C with an average temperature of 703°C for 1 to 1.5 hours. Following calcination, the tablets were observed to have an average relative crush strength of 12,2 kg (27 lb) measured by the same instrument used above to measure the uncalcined tablets.

The calcined tablets were then analyzed and found to contain $Fe_2O_3$—70.2 wt.%; $K_2O$—4.6 wt.%; $K_2CO_3$—10.6 wt.%; $Cr_2O_3$—2.8 wt.%; $V_2O_5$—4.0 wt.%; cement—3.3 wt.%; carbon—4.3 wt.%.

Examples and comparative runs

Eighteen experiments were made so that nine pairs of comparisons could be made between the catalytic performance and crush strength of catalyst tablets made by the method of the present invention and the catalytic performance and crush strength of catalyst extrudates made by conventional extrusion.

In each pair of experiments made, the catalyst pellets (tablets and extrudates) were made from the same formulation and had the same diameter. Three different formulations were used and three different diameters for the catalyst pellets were used for each formulation. Two different methods of pellet preparation were used for each diameter. The comparisons made are between those pairs of experiments in Table II whose catalyst pellets were made from the same formulation and have the same diameter.

In all of these experiments ethyl benzene (EB) was dehydrogenated to form styrene. Data are assembled in Table II.

The three different formulations used are well-known, useful dehydrogenation catalysts. The particular constituents of these formulations and their weight ratios (excluding free water) are given in Table I. These specific formulations are herein referred to as F-1, F-2, and F-3.

TABLE I
Slurry formulations (wt. percent)

| Catalyst | $Fe_2O_3$ | $Fe_2O_3 \cdot H_2O$ | $K_2CO_3 \cdot 3/2$ $H_2O$ | $K_2Cr_2O_7$ | $Cr_2O_3$ | $V_2O_5$ | Cement | Methyl Cellulose | Graphite |
|---|---|---|---|---|---|---|---|---|---|
| F-1 | 79.7 | — | 18.0 | — | 2.3 | — | — | — | — |
| F-2 | 50.6 | 12.6 | 17.9 | 2.7 | — | 2.7 | 3.0 | 8.9 | 1.6 |
| F-3 | 9.6 | 64.4 | 20.5 | 2.2 | — | — | 3.3 | — | — |

The three different pellet diameters chosen for testing each formulation were 0,32 cm (1/8 inch) 0,48 cm (3/16 inch) and 1,27 cm (1/2 inch). In the past, conventional catalyst pellet diameters have ranged from about 0,32 cm (1/8 inch) to about 0,64 cm (1/4 inch). It is known that smaller diameter pellets are normally more active and have greater crush strength than larger ones. Larger diameter pellets have the advantage of producing less pressure drop in the reactor, which allows the use of a lower absolute pressure than would be permitted otherwise. This in turn increases the conversion of the ethylbenzene. Thus a compromise has been utilized in the past between using a smaller catalyst pellet which has greater catalytic activity, and using a larger catalyst pellet which allows a lower operating pressure.

With the present invention such a compromise still has to be made, but by this invention, larger

5

**0014218**

catalyst pellets can be used with less loss in catalytic activity and crush strength as was experienced before when using conventionally made extruded pellets.

In the examples and comparative runs below pellet crush strength was determined on an average value of 20 pellets (tablets and extrudates) from each of the 18 batches of pellets made using a hardness tester instrument. This instrument gives a reading proportional to the total force, in units of pounds force (kg/force), required to crush the pellets.

The two different methods of catalyst preparation used for each diameter of each formulation were (1) the conventional method of extruding a paste into pellet extrudates and then calcining the extrudates, and (2) spray drying a slurry containing the catalyst constituents, sufficiently compressing the microspheres in a tabletting machine to form tablets of the same diameters and formulations as the extrudates, and then calcining the tablets. A detailed elaboration of these two methods for preparing the catalyst extrudates and tablets is given following Table II.

The extrudates and tabletted pellets prepared for each diameter of each formulation were tested for performance in mini-reactors. The reactor used for both the 0,32 cm (1/8 inch) and 0.48 cm (3/16 inch) extrudates and spray dried tablets was a part of a 0,89 m long (35 inch) 2,54 cm diameter (1 inch) 316 stainless steel pipe. The reactor used for the 1,27 cm (1/2 inch) diameter extrudates and spray dried tablets required 3,81 cm (1.5 inch) diameter pipe. Otherwise the mini-reactors were the same.

The pipes for both of these mini-reactors were erected upright. Both reactors were fitted wth a beaded wire heater and heater control means to control the temperature in their reactor zones and were well insulated. In every experiment, in either size reactor, the catalyst pellets were installed in the mini-reactors to form a catalyst bed 17,8 cm high (7 inches). Beneath the catalyst bed, but still in the pipe, 27,9 cm (11 inches) of spacers were installed to support the catalyst pellets. Above the catalyst bed, but still in the pipe 43,20 cm (17 inches) section of the pipe was used as a preheater and mixing zone. This zone contained porcelain saddles for column packing.

The preheater section also had a beaded wire heater wrapped around it as did the heater for the reactor section of the pipe described above.

Into this preheater there was fed 99 percent pure ethylbenzene (EB) and water. As the EB and water fed through the preheater stage they were heated to a temperature approaching reaction temperature. This temperature is above the vapor forming phase of the EB and the water. The porcelain saddles and boiling action of the EB and water caused the EB and water to become a well mixed vapor mixture. In this vapor state the mixture entered the reactor zone of the pipe in which were located the catalyst pellets (extrudates or tablets).

The liquid hourly space velocity (LHSV) (LHSV is defined as the volume flow of fluid per hour divided by the volume of catalyst present) of the EB-steam mixture was maintained at 1.0 vol/vol/hour. The weight ratio of the steam used to the EB used was maintained at 2.0:1.

For each batch of pellets tested, the operating temperature for the reactor was kept constant for each of the runs of a particular formulation, i.e., all the F-1 formulation experiments were made at 575°C, the F-2 at 610°C and the F-3 at 600°C. Conversion data are given in Table II.

TABLE II

Examples and Comparative Runs made at constant temperatures for each formulation

| Experiment No. | Formulation[1] | Pellet diameter | Type of pellet | Average crush strength, kg (lb) | Operating Temp. (°C) | % Conv. of EB to styrene |
|---|---|---|---|---|---|---|
| Comparative Run 1 | F-1 | 0.32 cm (1/8") | EX[2] | 8.6 (19) | 575 | 38.86 |
| Example 1 | F-1 | 0.32 cm (1/8") | SDT[3] | 16.8 (37) | 575 | 48.82 |
| Comparative Run 2 | F-1 | 0.48 cm (3/16") | EX | 5.9 (13) | 575 | 40.30 |
| Example 2 | F-1 | 0,48 cm (3/16") | SDT | 15.9 (35) | 575 | 41.14 |
| Comparative Run 3 | F-1 | 1.27 cm (1/2") | EX | 16.8 (37) | 575 | 9.13 |
| Example 3 | F-1 | 1.27 cm (1/2") | SDT | 16.8 (37) | 575 | 17.24 |
| Comparative Run 4 | F-2 | 0.32 cm (1/8") | EX | 9.1 (20) | 610 | 57.49 |
| Example 4 | F-2 | 0.32 cm (1/8") | SDT | 9.1 (20) | 610 | 61.24 |
| Comparative Run 5 | F-2 | 0.48 cm (3/16") | EX | 7.25 (16) | 610 | 50.39 |
| Example 5 | F-2 | 0.48 cm (3/16") | SDT | 13.6 (30) | 610 | 57.49 |
| Comparative Run 6 | F-2 | 1.27 cm (1/2") | EX | 16.3 (36) | 610 | 29.32 |
| Example 6 | F-2 | 1.27 cm (1/2") | SDT | 16.3 (36) | 610 | 35.64 |

6

TABLE II (contd.)
Examples and Comparative Runs made at constant temperatures for each formulation

| Experiment No. | Formulation[1] | Pellet diameter | Type of pellet | Average crush strength, kg (lb) | Operating Temp. (°C) | % Conv. of EB to styrene |
|---|---|---|---|---|---|---|
| Comparative Run 7 | F-3 | 0.32 cm (1/8″) | 55.38 | EX | 9.52 (21) | 600 |
| Example 7 | F-3 | 0.32 cm (1/8″) | 58.40 | SDT | 9.52 (21) | 600 |
| Comparative Run 8 | F-3 | 0.48 cm (3/16″) | 50.89 | EX | 2.72 (6) | 600 |
| Example 8 | F-3 | 0.48 cm (3/16″) | 56.44 | SDT | 7.25 (16) | 600 |
| Comparative Run 9 | F-3 | 1.27 cm (1/2″) | 33.13 | EX | 6.80 (15) | 600 |
| Example 9 | F-3 | 1.27 cm (1/2″) | 38.24 | SDT | 6.80 (15) | 600 |

[1]See Table 1 for formulation data for F-1, F-2, and F-3.
[2]Pellets made by conventional catalyst extrusion methods.
[3]Spray dried tablet made by the method of this invention.

From Table II it can be seen that the crush strength of the spray dried tablets prepared by the method of this invention was as good or better than that of the conventionally made extrudates for a given formulation and a given diameter. Further, it is seen that in every case the tablet made by the method of this invention produced a higher conversion of ethylbenzene to styrene.

The extrudate pellets of the three formulations used were made by the conventional extrusion method. This method is as follows:

First the soluble components used for making the extrudates for each particular batch were dissolved in water. The water-insoluble components were in powder form and were thoroughly mixed.

The water-soluble constituents of the formulations in Table I are $K_2CO_3 \cdot 3/2H_2O$, $K_2Cr_2O_7$, and $V_2O_5$.

The small particles of water-insoluble constituents of the formulation being used were then added to the solution of water-soluble constituents of that formulation and thoroughly mixed to form a slurry having about 75 weight percent solid material. This solid material percentage is calculated by considering both the soluble and insoluble material as solid.

Next the resulting mixed slurries were dried in an oven at 110°C until the free water content of each mixture was down to 10 to 12 weight percent so that a paste was formed.

The paste for each formulation and each of the three diameters used was then extruded using a commercial type pellet mill.

The extrudates formed by this pellet mill varied in length from 0,32 cm (1/8 inch) to 1,27 cm (1/2 inch) for each of the three diameter pellet extrudates produced, with the larger diameter pellets tending to have the longer lengths. These lengths were formed as the continuous extrusions of paste exiting from the pellet die was cut by a knife.

The pellet extrudates were then calcined at a temperature of about 700°C—900°C for 3 hours.

The different batches of spray dried tablets made for the comparative runs of Table II were made by first forming the constituents of the particular formulation into a water slurry in the same way the mixture used to form the paste for the conventional extrudate pellets above was made. Thus, the water-soluble constituents were added to water and this solution was thoroughly mixed with the water-insoluble components obtained from the same source from which the insoluble constituents were obtained. The slurry contained about 35 weight percent solid material. The weight percent of the solid material of the slurry was calculated as including the water-soluble as well as the water-insoluble constituents.

The slurry was fed to a laboratory disk type spray drier where it was led from a reservoir to the top of the atomizer wherein it was passed down a feed tube to an atomizer wheel. The slurry entered the interior of the wheel and because of the high rotational speed of the wheel (40,000 rpm), it is flung outward at a high rate of speed in droplet form. These droplets produced a very fine, uniform mist. This mist entered the drying chamber of the atomizer from the periphery of the atomizer wheel. The atomizer wheel had a diameter of 63 millimeters. The drying air at the drying chamber inlet was maintained at 210°C and the outlet temperature in the cyclone filter was maintained at 90°C. The feed rate to the atomizer was maintained at about 1000 grams/hour. The spray dried microspheres produced for all the experimental runs using spray dried tablets contained about 2 percent moisture. Their diameters ranged from 10 $\mu$m to 50 $\mu$m with most of the microspheres having a diameter close to 30 $\mu$m. Under an electron microscope some of these microspheres appeared to be of the hollow ruptured type.

These microspheres were then fed to a manually operated pellet press having dies with the same

**0014218**

inside diameter as that desired for the tablets, i.e., 0,32 cm (1/8 inch), 0,48 cm (3/16 inch) and 1,27 cm (1/2 inch). The dies used were filled with the spray dried microspheres. Sufficient amounts of microspheres were used in the dies so that the length and diameter of the tablets formed were the same. The tablets were cylindrical in shape with the ends which were slightly convex.

Following the tabletting of the spray dried microsphere the formed tablets were then calcined in the same way as were the conventionally made extrudates above, i.e., these spray dried tablets were heated at a temperature of 700°C to 900°C for about 3 hours.

The crush strength of these spray dried tablets used in Table II were measured following the calcining of the spray dried tablets.

## Claims

1. A method for making an unsupported fixed-bed catalyst tablet characterized by

(a) forming microspheres from a slurry of catalyst source constituents of the oxides of iron, potassium, and chromium by spray drying a slurry containing these catalyst source constituents;
(b) forming a catalyst tablet by subjecting a preselected amount of the microspheres to a compressive pressure of at least (208×10$^5$. Pa) (212 kg/cm$^2$); and
(c) calcining the tablet at a temperature greater than 500°C.

2. The catalyst table made by the process of Claim 1.

3. A method for making an unsupported fixed-bed catalyst tablet for use in the dehydrogenation of ethylbenzene to styrene characterized by

(a) spray drying a slurry into microspheres, with said microspheres having an average diameter of from 20 $\mu$m to 200 $\mu$m and a moisture content no greater than 5 weight percent, with said slurry being comprised of water and catalyst source constituents of the oxides of iron, potassium, and chromium, and with said slurry having a water content of from 20 weight percent to 70 weight percent;
(b) forming a substantially cylindrically shaped tablet from a preselected amount of said microspheres by subjecting these microspheres to a compressive pressure of at least (519×10$^5$ Pa) (1550 kg/cm$^2$), said tablet having a diameter of from 0.32 cm (1/8 inch) to 1.27 cm (1/2 inch); and
(c) calcining the tablet at a temperature of at least 500°C.

## Patentansprüche

1. Verfahren zur Herstellung einer trägerlosen Festbett-Katalysatortablette, gekennzeichnet durch:

(a) Formen von Mikrokügelchen aus einem Brei aus den Ausgangsbestandteilen des Katalysators, Eisen-, Kalium- und Chromoxiden, durch Sprühtrocknen eines Breis, der diese Katalysator-Ausgangsbestandteile enthält;
(b) Formen der Katalysatortablette durch Aussetzen einer vorgewählten Menge dieser Mikrokügelchen einem Kompressionsdruck von mindestens 208×10$^5$ Pa (212 kp/cm$^2$); und
(c) Kalzinieren der Tablette bei einer Temperatur von über 500°C.

2. Die durch das Verfahren gemäß Anspruch 1, hergestellte Katalysatortablette.

3. Verfahren zur Herstellung einer trägerlosen Festbett-Katalysatortablette zur Anwendung bei der Dehydrierung von Ethylbenzol zu Styrol, gekennzeichnet, durch;

(a) Sprühtrocknen eines Breis zu Mikrokügelchen, wobei diese Mikrokügelchen einen durchschnittlichen Durchmesser von 20—200 $\mu$m und einen Feuchtigkeitsgehalt von höchstens 5 Gewichtprozent haben und der genannte Brei aus Wasser und aus den Katalysatorausgangsbestandteilen Eisen-, Kalium- und Chromoxiden besteht und der genannte Brei einen Wassergehalt zwischen 20 und 70 Gewichtsprozent hat;
(b) Formen einer im wesentlichen zylindrischen Tablette aus einer vorgewählten Menge der genannten Mikrokügelchen durch Aussetzen dieser Mikrokügelchen einem Kompressionsdruck von mindestens 519×10$^5$ Pa (1550 kp/cm$^2$) wobei diese Tablette einen Durchmesser von 0,32 cm (1/8 Zoll) bis 1,27 cm (1/2 Zoll) hat; und
(c) Kalzinieren der Tablette bei mindestens 500°C.

## Revendications

1. Procédé de fabrication d'un comprimé de catalyseur non-supporté pour lit fixe, caractérisé par:

8

a) la formation de microsphères à partir d'une suspension de constituants des oxydes de fer, de potassium et de chrome formant la source du catalyseur, en séchant par pulvérisation la suspension contentant ces constituants formant la source du catalyseur;

b) la formation d'un comprimé de catalyseur en soumettant une quantité pré-sélectionnée des microsphères à une pression compressive d'au moins $208 \times 10^5$ Pa (212 kg/cm²); et

c) la calcination du comprimé à une température supérieure à 500°C.

2. Le comprimé de catalyseur fabriqué par le procédé de la revendication 1.

3. Procédé de fabrication d'un comprimé de catalyseur non-supporté pour lit fixe destiné à être utilisé dans la déshydrogénation de l'éthylbenzène en styrène, caractérisé par:

a) le séchage par pulvérisation d'une suspension en microsphères, ces microsphères ayant un diamètre moyen de 20 $\mu$m à 200 $\mu$m et un taux d'humidité ne dépassant pas 5% en poids, ladite suspension étant constituée par de l'eau et par des constituants des oxydes de fer, de potassium et de chrome formant la source du catalyseur, et ladite suspension ayant une teneur en eau de 20% en poids à 70% en poids;

b) la formation d'un comprimé ayant une forme sensiblement cylindrique à partir d'une quantité présélectionnée desdites microsphères en soumettant ces microsphères à une pression compressive d'au moins $519 \times 10^5$ Pa (1550 kg/cm²), ce comprimé ayant un diamètre de 0,32 cm (1/8 inch) à 1,27 cm (1/2 inch); et

c) la calcination du comprimé à une température d'au moins 500°C.